# EUROPÄISCHE PATENTSCHRIFT

(11) **EP 4 608 279 B1**
(45) Veröffentlichungstag und Bekanntmachung des Hinweises auf die Patenterteilung: **05.08.2026**
(21) Anmeldenummer: 23798366.3
(22) Anmeldetag: 25.10.2023
(51) Int. Cl.: A61B 10/00, A61B 5/20, G01F 1/00, G01F 3/38, G01F 23/02, G01F 23/263

(54) **MESSEINRICHTUNG UND VERFAHREN ZUR MESSUNG**
MEASURING DEVICE AND METHOD FOR MEASURING
DISPOSITIF DE MESURE ET PROCÉDÉ DE MESURE

(30) Priorität: 25.10.2022 EP 22203523
(43) Veröffentlichungstag der Anmeldung: 03.09.2025
(73) Patentinhaber: MEON Medical Solutions GmbH & Co KG, 8010 Graz (AT)
(72) Erfinder: BAUMGARTNER, Jürgen, 4600 Wels (AT); GLEINSER, Harald, 8010 Graz (AT); LEINER, Marco J.P., 8045 Graz (AT); RÜTHER, Horst, 8047 Hart bei Graz (AT)
(74) Vertreter: SONN Patentanwälte GmbH & Co KG
(86) Internationale Anmeldenummer: PCT/EP2023/079703
(87) Internationale Veröffentlichungsnummer: WO 2024/089062

(56) Entgegenhaltungen:
- CN-A- 110 226 939
- CN-A- 112 089 446
- DE-A1- 3 118 158
- US-A- 5 616 138
- US-B2- 11 006 879

## Beschreibung

Die Erfindung betrifft eine Messeinrichtung zur Messung der Ausscheidung einer Körperflüssigkeit, insbesondere von Urin, eines Patienten mit einem Katheter, aufweisend:
ein Gehäuse,
einen Messbehälter zur Aufnahme eines Messvolumens der Körperflüssigkeit,
eine Flüssigkeitsverbindung zum Zuführen des Messvolumens der Körperflüssigkeit von einem Ableitschlauch in den Messbehälter,
ein erstes Ventil zum Freigeben und Blockieren des Durchtritts der Körperflüssigkeit durch einen ersten Längsabschnitt des Ableitschlauchs,
ein zweites Ventil zum Freigeben und Blockieren des Durchtritts der Körperflüssigkeit durch einen zweiten Längsabschnitt des Ableitschlauchs,
eine Sensoreinheit zur Erfassung des Messvolumens der Körperflüssigkeit in dem Messbehälter.

Weiters betrifft die Erfindung ein Verfahren zur Messung der Ausscheidung einer Körperflüssigkeit, insbesondere von Urin, eines Patienten über einen Katheter.

Der allgemeine Stand der Technik wird durch DE3118158A1, CN110226939A, US5616138A1 gebildet.

Wie beispielsweise in der DE 31 18 158 A1 beschrieben wird, werden Urinsammelbeutel routinemäßig bei postoperativen Patienten sowie Patienten mit bestimmten urologischen Funktionsstörungen verwendet. Dabei wird der Patient zunächst katheterisiert und das andere Katheterende wird mit dem Sammelbehälter durch einen Schlauch verbunden. Vielfach wird der Beutel unterhalb des Patienten entweder am Bettgestell oder einer anderen Aufhängevorrichtung fixiert und der Urin fließt durch die Wirkung der Schwerkraft vom Patienten über den Katheter und die Schlauchverbindung in den Beutel. Die DE 31 18 158 A1 schlägt nun eine verbesserte Methode vor, mit welcher die Urinmenge und die Urindurchflussrate automatisch gemessen werden soll. Zu diesem Zweck wird eine kalibrierte Kammer zwischen dem zum Patienten führenden Katheter und dem Urinsammelbeutel angeordnet und an einem Kontrollgehäuse befestigt. Latexschläuche verbinden die Kammer mit dem Katheter an der Eingangs- und mit dem Urinsammelbeutel an der Ausgangsseite. Hahn- oder Ventilvorrichtungen sind am Gehäuse oberhalb und unterhalb der kalibrierten Kammer vorgesehen, um alternativ den Fluss am Eingang oder Ausgang zu schließen. Eine optische Sensoreinrichtung ist direkt oberhalb der kalibrierten Kammer und unterhalb der oberen Ventilvorrichtung angeordnet, um den Anstieg des Urinpegels innerhalb der kalibrierten Kammer zu überwachen und bei Erreichen eines vorher festgelegten Pegels, der durch die optische Abtasteinrichtung gegeben ist, eine Betätigung der unteren und oberen Ventileinrichtungen einzuleiten, sodass der Zufluss von Flüssigkeit in die Kammer unterbrochen und das festgelegte kalibrierte Volumen in den Sammelbeutel abgeleitet wird. Sensorvorrichtungen und externe Schaltkreise zeigen die Anzahl der Entleerungen der gefüllten Kammer an und multiplizieren diese mit dem Kammervolumen, um so ein digitales Ausgangssignal der gesamten Urinmenge durch die Kammer bereitzustellen. Durch Verwendung elektronischer Zeitgebervorrichtungen innerhalb des Systems ist eine getrennte digitale Anzeige der Urindurchflussrate vorhanden.

Dieser Stand der Technik bietet eine wesentliche Verbesserung gegenüber dem manuellen Ablesen der Urinmenge am Urinsammelbehälter. Allerdings bringt der Stand der Technik nach wie vor Nachteile mit sich. Die kalibrierte Messkammer wird zwischen einem oberen, zum Katheter führenden Schlauch und einem unteren, zum Urinsammelbehälter führenden Schlauch installiert. Nachteiligerweise muss das gesamte Urinvolumen durch die Messkammer geführt werden. Es besteht keine Möglichkeit, einen Teil des Urinstroms selektiv an der Messkammer vorbei zu führen. Weiters wird eine Nachrüstung bestehender Kathetersysteme erschwert, weil der Ableitschlauch für die Ankopplung des Messsystems durchtrennt und beide Enden einzeln mit dem Messsystem verbunden werden müssen.

Demgegenüber besteht die Aufgabe der vorliegenden Erfindung darin, zumindest einzelne Nachteile des Standes der Technik zu lindern bzw. zu beheben. Diese Aufgabe wird durch eine Messeinrichtung nach Anspruch 1 und ein Verfahren nach Anspruch 10 gelöst. Bevorzugte Ausführungsformen sind in den abhängigen Ansprüchen angegeben.

Bei der erfindungsgemäßen Messeinrichtung ist das Gehäuse seitlich zu einer Längsführung für den Ableitschlauch versetzt angeordnet, wobei die Flüssigkeitsverbindung zur Abzweigung des Messvolumens der Körperflüssigkeit zwischen dem ersten und dem zweiten Ventil vom Ableitschlauch in den Messbehälter eingerichtet ist.

Für die Zwecke dieser Offenbarung beziehen sich die Orts- und Richtungsangaben, wie "horizontal", "vertikal", "oben", "unten", auf den bestimmungsgemäßen Gebrauchszustand der Messeinrichtung bei vertikaler Ausrichtung des Ableitschlauchs entlang des Gehäuses. Selbstverständlich kann das Gehäuse auch anders positioniert werden, wobei dann die Orts- und Richtungsangaben entsprechend zu übertragen sind.

Somit ist der Messbehälter der erfindungsgemäßen Messeinrichtung seitlich, d.h. in Richtung senkrecht zur Längsrichtung der Längsführung, von der Längsführung beabstandet. Dadurch erstreckt sich der Messbehälter zumindest abschnittsweise, vorzugsweise im Wesentlichen vollständig, neben der Längsführung, in welcher im montierten Gebrauchszustand der Ableitschlauch angeordnet ist. Vorzugsweise sind die Längsführung und der Ableitschlauch entsprechend ausgestaltet, so dass der Ableitschlauch im montierten Gebrauchszustand in der Längsführung seitlich fixiert ist. Die erfindungsgemäße Ausführung bringt mehrere Vorteile mit sich. Durch die seitliche Anordnung des Messbehälters können das erste und das zweite Ventil am Ableitschlauch näher als beim Stand der Technik der DE 31 18 158 A1 aneinanderrücken. Bevorzugt ist der Längsabstand zwischen dem ersten dem zweiten Ventil geringer, insbesondere um ein Mehrfaches geringer, als die maximale Füllstandshöhe der Körperflüssigkeit innerhalb des Messbehälters. Demgegenüber musste beim Stand der Technik das eine Ventil oberhalb des Messbehälters und das andere Ventil unterhalb des Messbehälters vorgesehen werden. Erfindungsgemäß setzt zwischen dem ersten und dem zweiten Ventil die beispielsweise einen Schlauch oder ein Rohr aufweisende Flüssigkeitsverbindung an, über welche die Körperflüssigkeit, insbesondere Urin, vom insbesondere mit einem Katheter verbundenen Ableitschlauch abgezweigt wird. In der Offenstellung des ersten und in der Schließstellung des zweiten Ventils kann die Körperflüssigkeit vom Ableitschlauch über die Flüssigkeitsverbindung in den Aufnahmeraum im Inneren des Messbehälters eingeleitet werden. Beim Stand der Technik fließt die gesamte Flüssigkeit vom Ableitschlauch in den Messbehälter. Erfindungsgemäß ist hingegen die Flüssigkeitsverbindung zur Abzweigung des Messvolumens eingerichtet, so dass nicht zwangsläufig die gesamte Flüssigkeit durch den Messbehälter fließen muss. Beispielsweise kann die Körperflüssigkeit, etwa ein Vorlauf, ohne Messung am Messbehälter vorbeigeführt werden. Dafür kann das erste und das zweite Ventil in die Offenstellung geschalten werden. Somit kann lediglich jene Flüssigkeitsmenge, die tatsächlich gemessen werden soll, über die Flüssigkeitsverbindung in den Messbehälter abgezweigt werden. Weiters kann der Zusammenbau der Messeinrichtung wesentlich erleichtert werden. Auch kann eine Ausführung ermöglicht werden, bei welcher die Messeinrichtung mit einem bestehenden Urindrainagesystem verbunden werden kann.

Somit können die folgenden Betriebszustände unterschieden werden.

Ist das erste Ventil in der Offenstellung und das zweite Ventil in der Schließstellung angeordnet, wird der Messbehälter befüllt. Ist das zweite Ventil in der Offenstellung angeordnet, wird der Messbehälter entleert. Sind das erste Ventil und das zweite Ventil in der Offenstellung angeordnet, kann die Körperflüssigkeit, etwa ein Vorlauf, durch den Ableitschlauch am Messbehälter vorbeigeführt werden, so dass die Körperflüssigkeit nicht durch den Messbehälter strömt.

Bei einer besonders bevorzugten Ausführungsform ist die Flüssigkeitsverbindung zudem zum Ableiten des Messvolumens der Körperflüssigkeit vom Messbehälter zurück in den Ableitschlauch eingerichtet. Vorteilhafterweise kann der Messbehälter bei dieser Ausführungsform über dieselbe Flüssigkeitsverbindung, welche insbesondere ein Rohr oder ein Schlauch ist, befüllt und entleert werden. Dadurch kann eine erhebliche Vereinfachung erzielt werden. Weiters kann die Gefahr von Undichtigkeiten reduziert werden. Vorteilhaft ist weiters, dass die Ankopplung eines kommerziell verfügbaren Drainagesystems (ohne Messsystem) erleichtert wird.

Weiters ist bevorzugt, wenn die Flüssigkeitsverbindung mit einer Eintrittsöffnung am unteren Bereich des Messbehälters, insbesondere am Boden, d.h. am unteren Ende, des Messbehälters verbunden ist. Eine solche Befüllung von unten nach oben erlaubt das Entweichen von Luft aus dem Messvolumen. Damit wird die Messgenauigkeit erhöht. Weiters kann der Messbehälter so über ein und dieselbe Flüssigkeitsverbindung zum Ableitschlauch befüllt und entleert werden. Dafür ist es günstig, wenn die Flüssigkeitsverbindung unterhalb einer Marke für den unteren Flüssigkeitspegel in den Messbehälter mündet. Besonders bevorzugt mündet die Flüssigkeitsverbindung in den Boden des Messbehälters. Dadurch kann eine vollständige Entleerung des Messbehälters erreicht werden.

Als erstes (oberes) Ventil ist bevorzugt ein erstes (oberes) Quetschventil und/oder als zweites (unteres) Ventil ist bevorzugt ein zweites (unteres) Quetschventil vorgesehen. Das erste bzw. zweite Ventil kann mit einem ersten bzw. zweiten Antrieb zwischen der Schließ- und Offenstellung bewegt werden.

In einer Ausführungsform weist die Messeinrichtung den Ableitschlauch auf, wobei der Ableitschlauch über die Flüssigkeitsverbindung mit dem Messbehälter verbunden ist. In einer anderen Ausführungsform ist die Messeinrichtung zur Verbindung der Flüssigkeitsverbindung mit dem (nicht Teil der Messeinrichtung bildenden) Ableitschlauch eingerichtet. Bei dieser Ausführung wird der Ableitschlauch vor Gebrauch, beispielsweise in einem Krankenhaus, mit der Messeinrichtung verbunden.

In Gebrauch ist der Ableitschlauch bevorzugt zumindest seitlich, d.h. senkrecht zur Längsrichtung des Ableitschlauchs, in der Längsführung fixiert. Die Längsführung verläuft bevorzugt geradlinig durch das Gehäuse. Bevorzugt endet die Längsführung einerseits an einer oberen Stirnseite des Gehäuses und andererseits an einer unteren Stirnseite des Gehäuses.

Bei einer bevorzugten Ausführungsform ist die Längsführung für eine reversibel lösbare, d.h. ohne Beschädigung wiederholt lösbare, Anordnung des Ableitschlauchs eingerichtet.

Bei einer weiteren bevorzugten Ausführungsform ist der Messbehälter reversibel lösbar mit dem Gehäuse verbunden. Zu diesem Zweck kann das Gehäuse eine Ausnehmung aufweisen, in welcher der Messbehälter reversibel lösbar angeordnet ist, beispielsweise über eine insbesondere werkzeuglos herstellbare und lösbare Steckverbindung. Im montierten Zustand ist der Messbehälter in der Ausnehmung fixiert. Diese Ausführungsform hat den Vorteil, dass der Messbehälter als Einmal-Artikel ausgeführt werden kann, welcher nach Gebrauch vom Gehäuse entfernt und durch einen neuen Messbehälter ersetzt werden kann. Bevorzugt ist die Ausnehmung des Gehäuses so ausgeführt und angeordnet, dass der Messbehälter nur teilweise, d.h. nicht vollständig, vom Grundkörper umschlossen ist, so dass der Füllstand des Messbehälters von außen mit dem Auge sichtbar ist. Um die Sichtbarkeit zu erhöhen, kann die Messeinrichtung eine Lichtquelle, beispielsweise ein LED-Licht, aufweisen.

Bei einer bevorzugten Ausführungsform ist die Sensoreinheit zur Bestimmung eines oberen Flüssigkeitspegels und/oder eines unteren Flüssigkeitspegels der Körperflüssigkeit innerhalb des Messbehälters eingerichtet. Bevorzugt sind der Messbehälter und die Sensoranordnung so kalibriert, dass das Flüssigkeitsvolumen zwischen dem oberen und dem unteren Flüssigkeitspegel bekannt ist. Bei einer bevorzugten Ausführungsform weist die Sensoreinheit zumindest einen kapazitiven Sensor auf.

Bei einer bevorzugten Ausführungsform ist die Sensoreinheit mit einer Steuereinheit zum Umschalten jeweils des ersten und des zweiten Ventils zwischen der Offen- und der Schließstellung verbunden.

Ist das zweite (untere) Ventil geschlossen und das erste (obere) Ventil geöffnet (Füllzustand), kann der Messbehälter durch die über den Katheter nachfließende Körperflüssigkeit gefüllt werden, bis die Sensoreinheit den oberen Flüssigkeitspegel detektiert oder mit einer Zeitgebereinrichtung eine verstrichene Zeit festgestellt wird. Daraufhin wird über die Steuereinheit das obere Ventil geschlossen und das untere Ventil geöffnet (Entleerzustand), wodurch die Ableitung der im Messbehälter befindlichen Körperflüssigkeit über die Flüssigkeitsverbindung zurück in den Ableitschlauch und bevorzugt von diesem in den tiefer gelegenen Sammelbehälter, z.B. in einen Urinsammelbeutel, bewirkt wird. Detektiert die Sensoreinheit den unteren Flüssigkeitspegel, kann die Steuereinheit das zweite Ventil schließen und das erste Ventil öffnen. Dieser Vorgang kann beliebig oft wiederholt werden.

Bei einer weiteren bevorzugten Ausführungsform ist die Steuereinheit mit einer Auswerteeinheit verbunden. Mit der Betätigung des ersten bzw. zweiten Ventils kann von der Steuereinheit ein Signal an die Auswerteeinheit gesendet werden, mit welchem die in den Sammel- bzw. Auffangbehälter über die Zeit abgegebene Flüssigkeitsmenge angezeigt und/oder aufgezeichnet wird.

Bei einer weiteren bevorzugten Ausführungsform ist die Sensoreinheit zudem dazu eingerichtet, zusätzlich zu dem unteren und dem oberen Flüssigkeitspegel auch zumindest einen, vorzugsweise mehrere, dazwischenliegende Flüssigkeitspegel zu erfassen. Die Sensoreinheit kann, vorzugsweise laufend und insbesondere über die Steuereinheit, entsprechende Signale an die Auswerteeinheit übermitteln. Derart kann die Auswerteeinheit die ausgeschiedene Menge an Flüssigkeit und die Flussrate wesentlich genauer bestimmen. Dies ist insbesondere bei niedrigen Flussraten vorteilhaft. Zu diesem Zweck kann die Sensoreinheit einen Kapazitätsmessstreifen, insbesondere entlang des Messbehälters, aufweisen.

Bei einer weiteren bevorzugten Ausführungsform ist vorgesehen, dass zumindest ein weiterer Sensor, beispielsweise eine Lichtschranke oder ein kapazitiver Sensor, benachbart der Längsführung für den Ableitschlauch vorgesehen ist. Dieser weitere Sensor ist bevorzugt dazu eingerichtet, zu überprüfen, ob sich in den jeweiligen Längsabschnitten des Ableitschlauchs Luft oder Flüssigkeit befindet. Entspricht zu einem gegebenen Betriebszeitpunkt der vorbeiströmende Inhalt (Gas bzw. Flüssigkeit) der einzelnen Schlauchabschnitte nicht dem Soll, kann, je nach Ausführung, ein Alarmsignal abgegeben werden und/oder das erste bzw. zweite Ventil geöffnet werden.

Zur einfachen und schnellen Verbindung weist die Längsführung bevorzugt eine Einführöffnung zum Einführen des Ableitschlauchs auf, wobei sich die Einführöffnung bevorzugt über im Wesentlichen die gesamte Länge der Längsführung erstreckt. Die Einführöffnung ist bevorzugt als Längsschlitz ausgebildet, welcher sich an einer Längsseite des Gehäuses erstrecken kann. Vorteilhafterweise kann der Ableitschlauch mittels temporärer elastischer Verformung durch den Längsschlitz gequetscht und so zumindest teilweise innerhalb der Längsführung angeordnet werden. Im montierten Zustand wird die elastische Verformung des Ableitschlauchs zurückgebildet, wodurch der Ableitschlauch zuverlässig in der Längsführung gehalten wird. Bei dieser Ausführungsform kann die Längsführung eine im Querschnitt (senkrecht zur Längsrichtung der Längsführung gesehen) im Wesentlichen kreisbogenförmige Führungs- und Haltefläche aufweisen, an welche die Einführöffnung anschließt. Um ein unbeabsichtigtes Herauslösen des Ableitschlauchs aus der Längsführung zu verhindern, überstreicht die im Querschnitt im Wesentlichen kreisbogenförmige Führungsfläche einen Mittelpunktswinkel von mehr als 180°.

Bei einer bevorzugten Ausführungsform erstreckt sich die Längsachse des Messbehälters im Wesentlichen parallel zur Längsachse der Längsführung. Dadurch verläuft die Längsführung längs neben dem Messbehälter. In Gebrauch strömt die Körperflüssigkeit durch den in der Längsführung angeordneten Ableitschlauch abschnittsweise benachbart des Messbehälters, d.h. auf derselben Höhe, aber seitlich versetzt.

Bevorzugt ist ein zylindrischer Messbehälter vorgesehen, wobei sich die Längsachse des zylindrischen Messbehälters bevorzugt im Wesentlichen parallel und in einem seitlichen Abstand zur Längsachse der Längsführung erstreckt. Bevorzugt befindet sich der Messbehälter innerhalb des Gehäuses.

Um das Messvolumen vom Ableitschlauch über die Flüssigkeitsverbindung, vorzugsweise von unten, in den Messbehälter abzuzweigen, können verschiedene Ausführungsformen der Flüssigkeitsverbindung vorgesehen sein.

Bei einer ersten bevorzugten Ausführungsform ist der Ableitschlauch als Teil der Messeinrichtung vorhanden. Der Ableitschlauch, die Flüssigkeitsverbindung und der Messbehälter sind als Messeinheit ausgebildet, welche über eine reversibel lösbare Verbindung, insbesondere eine Steckverbindung, mit dem Gehäuse verbunden ist. Bei dieser Ausführung können der Ableitschlauch und die Flüssigkeitsverbindung unlösbar, d.h. nicht reversibel lösbar, beispielsweise einteilig, miteinander verbunden sein. Je nach Ausführung kann die Flüssigkeitsverbindung etwa als Abzweigung des Ableitschlauchs ausgebildet sein. Darüber hinaus können bei dieser Ausführung die Flüssigkeitsverbindung und der Messbehälter unlösbar, d.h. nicht reversibel lösbar, beispielsweise einteilig, miteinander verbunden sein. Besonders bevorzugt ist eine Ausführung, bei welcher der Ableitschlauch, die Flüssigkeitsverbindung und der Messbehälter unlösbar, d.h. nicht reversibel lösbar, beispielsweise einteilig, miteinander verbunden sind. Vor Gebrauch wird die Messeinheit reversibel lösbar mit dem Gehäuse verbunden, insbesondere durch Anordnung des Messbehälters in der Ausnehmung, des Ableitschlauchs in der Längsführung und/oder der Flüssigkeitsverbindung in einer Aussparung des Gehäuses, bevorzugt jeweils über eine Steckverbindung.

Bei einer zweiten bevorzugten Ausführungsform ist die Flüssigkeitsverbindung zur reversibel lösbaren Verbindung mit dem Ableitschlauch, insbesondere mit einem T-Stück zwischen dem ersten und zweiten Längsabschnitt des Ableitschlauchs, eingerichtet. Bei dieser Ausführung kann die Flüssigkeitsverbindung, beispielsweise ein Flüssigkeitsschlauch der Flüssigkeitsverbindung, ein Verbindungsstück aufweisen, welches insbesondere über das T-Stück mit dem ersten und dem zweiten Längsabschnitt des Ableitschlauchs verbunden werden kann. Die Verbindung zwischen der Flüssigkeitsverbindung und dem Ableitschlauch kann bei dieser Ausführung durch den Anwender, beispielsweise mittels Muffenverbindern oder Luer-Verbindern, erfolgen.

Bei einer dritten bevorzugten Ausführungsform weist die Flüssigkeitsverbindung ein Einstechelement auf, welches zum Einstechen des Ableitschlauchs, insbesondere im Wesentlichen senkrecht zur Längsführung, zwischen dem ersten und dem zweiten Ventil eingerichtet ist. Das Einstechelement kann eine Spitze aufweisen, welche zumindest eine Schlauchwand, je nach Ausführung genau eine Schlauchwand oder zwei gegenüberliegende Schlauchwände, durchdringen kann. Das Einstechelement ist bevorzugt aus einem starren Material, insbesondere Kunststoff oder Metall, gefertigt. Bei der Montage kann durch Ausübung von Druck der Ableitschlauch ein- oder beidseitig derart vom Einstechelement durchstochen werden, dass über eine seitliche Öffnung oder eine endseitige Öffnung des Einstechelements eine fluidische Verbindung zum Inneren des Ableitschlauchs hergestellt wird. Diese Art der Verbindung weist den besonderen Vorteil auf, dass auch bereits im Handel befindliche Drainagesysteme verwendet werden können, die konzeptionell nicht zur Ankopplung an die Messeinrichtung ausgelegt sind.

Bei einer bevorzugten Variante sind der Messbehälter und die Flüssigkeitsverbindung unlösbar, insbesondere einteilig, miteinander verbunden, wobei das vom Messbehälter abgewandte Ende der Flüssigkeitsverbindung als Einstechelement ausgebildet ist.

Bei einer bevorzugten Variante ist eine Schubvorrichtung vorgesehen, welche dazu eingerichtet ist, den in der Längsführung angeordneten Ableitschlauch gegen das währenddessen unbeweglich angeordnete Einstechelement zu drücken, um die fluidische Verbindung vom Ableitschlauch über die Flüssigkeitsverbindung zum Messbehälter herzustellen. Die Schubvorrichtung kann ein Schubelement aufweisen, welches von einer zurückgezogenen in eine vorgeschobene Stellung bewegt werden kann. Zu diesem Zweck weist die Schubvorrichtung bevorzugt einen Hebelmechanismus auf, welcher zwischen einer ersten Position entsprechend der zurückgezogenen Stellung des Schubelements und einer zweiten Position entsprechend der vorgeschobenen Stellung des Schubelements beweglich ist. Bevorzugt weist der Hebelmechanismus einen Handgriff auf, welcher vom Benutzer erfasst werden kann, um den Hebelmechanismus von der ersten in die zweite Position (und umgekehrt) zu bringen. Durch Betätigen des Hebelmechanismus wird das Schubelement so weit vorgeschoben, dass der Ableitschlauch gegen das Einstechelement gedrückt und dadurch aufgestochen wird. Im eingestochenen Zustand kann die Körperflüssigkeit vom Ableitschlauch über das Einstechelement in den Messbehälter abgezweigt werden.

Zur Erzielung möglichst unverfälschter Messwerte ist es günstig, wenn der Messbehälter, insbesondere an der Oberseite, ein Belüftungsventil aufweist. Das Belüftungsventil ist vorzugsweise als hydrophobe Belüftungsmembran ausgeführt. Das Belüftungsventil garantiert die Entlüftung des Messbehälters bei Füllung und die Belüftung des Messbehälters bei Entleerung. Die Belüftungsmembran ist vorzugsweise selbstsperrend und blockiert bei Benetzung mit Körperflüssigkeit. Vorzugsweise regeneriert die Belüftungsmembran nach Benetzung. Die Belüftungsmembran kann beispielsweise aus Polytetrafluorethylen (PTFE) gefertigt sein.

Das erfindungsgemäße Verfahren zur Messung der Ausscheidung einer Körperflüssigkeit, insbesondere von Urin, eines Patienten, insbesondere mit einem Katheter, weist zumindest die folgenden Schritte auf:
Verbinden einer Messeinrichtung in einer der oben geschilderten Ausführungsformen mit einem, insbesondere vom Katheter wegführenden, Ableitschlauch, wobei der Ableitschlauch in der Längsführung des Gehäuses angeordnet wird,
Schalten des ersten Ventils in eine Offenstellung und des zweiten Ventils in eine Schließstellung,
Abzweigen eines Messvolumens der Körperflüssigkeit vom Ableitschlauch über die Flüssigkeitsverbindung in den Messbehälter,
Erfassung des Messvolumens der Körperflüssigkeit in dem Messbehälter, vorzugsweise zudem
Erfassen der Zeit, in welcher das Messvolumen in dem Messbehälter gesammelt wird.

Bei einer bevorzugten Ausführungsform weist das Verfahren zudem die folgenden Schritte auf:
Schalten des ersten Ventils in die Schließstellung und des zweiten Ventils in die Offenstellung,
Entleeren des Messvolumens der Körperflüssigkeit über die Flüssigkeitsverbindung. Auf diese Weise kann das Messvolumen in den Ableitschlauch zurückgeführt und von dort in einen Sammelbehälter, beispielsweise einen Urinsammelbeutel, abgeleitet werden.

Bei einer bevorzugten Ausführungsform wird das Flüssigkeits-Zeit-Volumen, insbesondere das Urin-Zeit-Volumen, bestimmt. Dafür ist folgendes Verfahren zur Messung der Ausscheidung der Körperflüssigkeit, insbesondere von Urin, eines Patienten, insbesondere mit einem Katheter, vorgesehen:
i. Verbinden einer Messeinrichtung in einer der oben geschilderten Ausführungsformen mit einem, insbesondere vom Katheter wegführenden, Ableitschlauch, wobei der Ableitschlauch in der Längsführung des Gehäuses angeordnet wird,
ii. Schalten des ersten Ventils in eine Offenstellung und des zweiten Ventils in eine Schließstellung,
iii. Abzweigen eines Messvolumens der Körperflüssigkeit vom Ableitschlauch über die Flüssigkeitsverbindung in den Messbehälter,
iv. Erfassung des Messvolumens der Körperflüssigkeit in dem Messbehälter,
v. Schalten des ersten Ventils in die Schließstellung und des zweiten Ventils in die Offenstellung,
vi. Entleeren des Messvolumens der Körperflüssigkeit über die Flüssigkeitsverbindung, und
vii. Wiederholen der Schritte ii. bis vi.,
viii. Bestimmen eines Flüssigkeits-Zeit-Volumens aus der wiederholten Erfassung des Messvolumens der Körperflüssigkeit in dem Messbehälter. Unter dem Flüssigkeits-Zeit-Volumen wird die in einem bestimmten Zeitintervall, beispielsweise binnen 24 Stunden, vom Patienten ausgeschiedene Körperflüssigkeit, insbesondere Urin, verstanden.

Weiters kann die Körperflüssigkeit, beispielsweise ein Vorlauf, ohne Befüllung des Messbehälters durch den Ableitschlauch abgeleitet werden. Zu diesem Zweck werden folgende Schritte durchgeführt:
Schalten des ersten Ventils in eine Offenstellung und Schalten des zweiten Ventils in eine Offenstellung,
Ableiten der Körperflüssigkeit durch den Ableitschlauch vorbei an dem Messbehälter, so dass der Messbehälter nicht befüllt wird.

Besonders bevorzugt ist es, wenn das Messvolumen der Körperflüssigkeit über eine Eintrittsöffnung am unteren Bereich, insbesondere am Boden, des Messbehälters eingebracht wird. Bevorzugt ist die Eintrittsöffnung derart am Messbehälter angeordnet, dass der Messbehälter über die Flüssigkeitsverbindung vollständig entleert werden kann.

Die Erfindung wird nachstehend anhand von in den Zeichnungen dargestellten Ausführungsbeispielen weiter erläutert.
Fig. 1 zeigt schematisch einen Längsschnitt entlang der Linie I-I in Fig. 2 einer Ausführungsform der erfindungsgemäßen Messeinrichtung zur Erfassung eines Urindurchflusses.
Fig. 2 zeigt einen Querschnitt der Messeinrichtung entlang der Linie II-II in Fig. 1.
Fig. 3 zeigt schematisch einen Längsschnitt entlang der Linie III-III in Fig. 4 einer weiteren Ausführungsform der erfindungsgemäßen Messeinrichtung.
Fig. 4 zeigt einen Querschnitt der Messeinrichtung entlang der Linie IV-IV in Fig. 3.
Fig. 5 zeigt schematisch einen Längsschnitt entlang der Linie V-V in Fig. 6 einer weiteren Ausführungsform der erfindungsgemäßen Messeinrichtung.
Fig. 6 zeigt einen Querschnitt der Messeinrichtung entlang der Linie VI-VI in Fig. 5.
Fig. 7 zeigt schematisch einen Längsschnitt entlang der Linie VII-VII in Fig. 8 einer weiteren Ausführungsform der erfindungsgemäßen Messeinrichtung.
Fig. 8 zeigt einen Querschnitt der Messeinrichtung entlang der Linie VIII-VIII in Fig. 7.
Fig. 9, Fig. 10 und Fig. 11A, Fig. 11B zeigen jeweils Details von Ausführungsformen der erfindungsgemäßen Messeinrichtung.
Fig. 12 bis 14 zeigen eine weitere Ausführungsform der Erfindung.

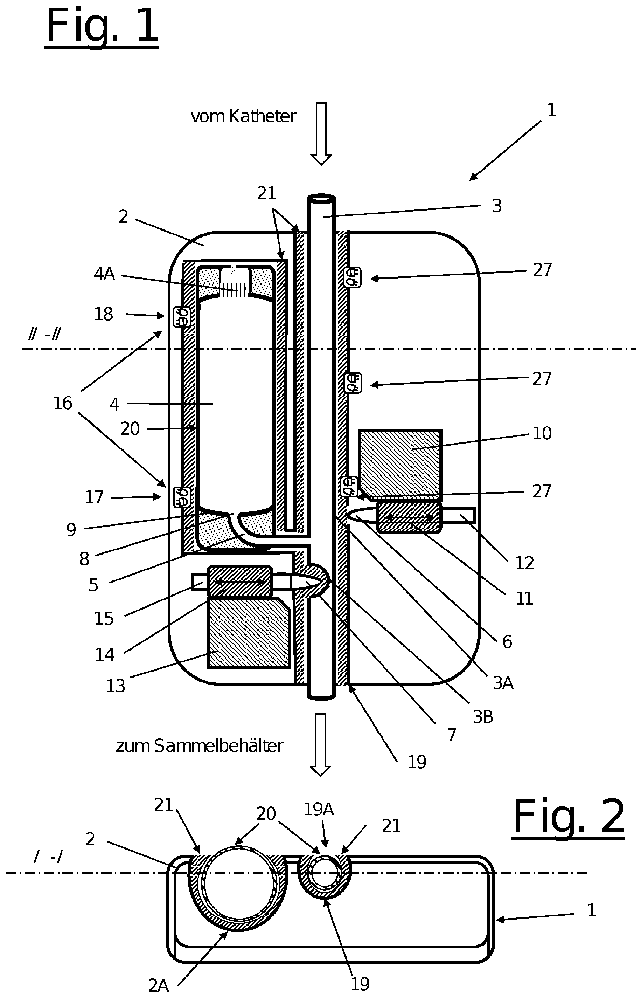

Fig. 1 und Fig. 2 zeigen eine erste Ausführungsform einer Messeinrichtung 1 zur Messung der Ausscheidung einer Körperflüssigkeit, insbesondere von Urin, eines Patienten. Mit Hilfe der Messeinrichtung 1 können insbesondere die Gesamtmenge und/oder die Durchflussrate einer mittels Drainage vom Patienten abgeleiteten Körperflüssigkeit, hier Urin, bestimmt werden. Besonders vorteilhaft ist eine Anwendung der Messeinrichtung 1 zur Bestimmung des Urin-Zeit-Volumens. Darunter wird die Menge an Urin verstanden, die in einem bestimmten Zeitintervall, beispielsweise binnen 24 Stunden, vom Patienten ausgeschieden wird. Die Messeinrichtung 1 weist einen Grundkörper, im Folgenden als Gehäuse 2 bezeichnet, auf, welches mit einem Ableitschlauch 3 verbunden ist, der an die Drainage, insbesondere an einen Katheter, angeschlossen ist (vgl. den mit "vom Katheter" bezeichneten Pfeil in Fig. 1). An dem Gehäuse 2 ist ein Messbehälter 4 angeordnet, welcher einen Aufnahmeraum zur Aufnahme eines Messvolumens der Körperflüssigkeit einschließt. Die Messeinrichtung 1 weist zudem eine Flüssigkeitsverbindung 5 auf, mit welcher selektiv ein Messvolumen der Körperflüssigkeit vom Ableitschlauch 3 in den Aufnahmeraum des Messbehälters 4 abgezweigt werden kann. Mit Hilfe eines ersten (oberen) Ventils 6 kann der Durchtritt der Körperflüssigkeit durch einen ersten (oberen) Längsabschnitt 3A des Ableitschlauchs selektiv freigegeben und blockiert werden. Der Durchtritt der Körperflüssigkeit durch einen zweiten (unteren) Längsabschnitt 3B des Ableitschlauchs 3 kann mit Hilfe eines zweiten (unteren) Ventils 7 selektiv freigegeben und blockiert werden. Das zweite Ventil 7 ist, bezogen auf den gezeigten vertikalen Gebrauchszustand des Gehäuses 2, unterhalb des ersten Ventils 6 angeordnet. Die Flüssigkeitsverbindung 5 zweigt (in Längsrichtung des Ableitschlauchs 3 gesehen) zwischen dem ersten 6 und dem zweiten Ventil 7 vom Ableitschlauch 3 ab. Vom Ableitschlauch 3 führt die Flüssigkeitsverbindung 5 in eine Eintrittsöffnung 8 an einem unterseitigen Boden 9 des Messbehälters 4. Somit wird der Messbehälter 4 von unten nach oben, gegen die Wirkung der Schwerkraft, befüllt. An der Oberseite weist der Messbehälter 4 ein Belüftungsventil 4A auf. In der gezeigten Ausführung ist als erstes Ventil 6 ein erstes Schlauchquetschventil und als zweites Ventil 7 ein zweites Schlauchquetschventil vorgesehen. Das erste Ventil 6 weist einen ersten Antrieb 10, beispielsweise einen ersten Servomotor, auf, welcher, vorzugsweise über ein erstes Getriebe 11, mit einem ersten Ventilkörper 12 verbunden ist. Zum Überführen des ersten Ventils 6 von der in Fig. 1 gezeigten Offenstellung in die Schließstellung wird der erste Ventilkörper 12 von außen gegen den ersten Längsabschnitt 3A des Ableitschlauchs 3 gedrückt (vgl. den Doppelpfeil in Fig. 1), wodurch der Durchfluss der Körperflüssigkeit an dieser Stelle unterbrochen wird. Entsprechend weist das zweite Ventil 7 einen zweiten Antrieb 13, beispielsweise einen zweiten Servomotor, auf, welcher, vorzugsweise über ein zweites Getriebe 14, mit einem zweiten Ventilkörper 15 verbunden ist. Zum Überführen des zweiten Ventils 7 von der in Fig. 1 gezeigten Schließstellung in die Offenstellung wird der zweite Ventilkörper 15 nach außen, vom zweiten Längsabschnitt 3B des Ableitschlauchs 3 weg, bewegt, wodurch der Durchfluss der Körperflüssigkeit an dieser Stelle freigegeben wird.

Gemäß Fig. 1 ist das erste Ventil 6 in der Offen- und das zweite Ventil 7 in der Schließstellung angeordnet (Füllzustand). Durch den Druck der nachfließenden Körperflüssigkeit wird das Messvolumen über die Flüssigkeitsverbindung 5 durch die Eintrittsöffnung 8 ins Innere des Messbehälters 4 gedrückt. Durch Umschalten des ersten 6 und zweiten Ventils 7 wird der Messbehälter 4, ebenfalls über die Flüssigkeitsverbindung 5, in den Ableitschlauch 3 entleert (vgl. Pfeil "zum Sammelbehälter" in Fig. 1).

Die Messeinrichtung 1 weist eine Sensoreinheit 16 auf, welche zur Erfassung des Messvolumens der Körperflüssigkeit in dem Messbehälter 4 eingerichtet ist. In der gezeigten Ausführung weist die Sensoreinheit 16 einen ersten (unteren) Sensor 17 zur Detektion eines den entleerten Zustand signalisierenden unteren Flüssigkeitspegels innerhalb des Messbehälters 4 und einen den befüllten Zustand signalisierenden zweiten (oberen) Sensor 18 zur Detektion eines oberen Flüssigkeitspegels innerhalb des Messbehälters 4 auf. Das Flüssigkeitsvolumen zwischen dem unteren und dem oberen Flüssigkeitspegel ist, insbesondere durch Kalibration, bekannt. Zur laufenden Bestimmung des Füllstands des Messbehälters 4 können aus dem Stand der Technik an sich bekannte Sensoren 17, 18 verwendet werden. Als Sensoren 17, 18 können beispielsweise Lichtschranken, insbesondere mit LEDs und Photodioden, herangezogen werden, die den Flüssigkeitspegel im Messbehälter 4 durch eine Transmissions- oder Reflexionsmessung bestimmen können. Bevorzugt werden zur Bestimmung des Füllstands kapazitive Sensoren verwendet. In der Ausführungsform der Fig. 1 sind als kapazitive Sensoren Kapazitätselektroden vorgesehen, mit welchen ein unteres und ein oberes Flüssigkeitsniveau erfasst wird. Das Kapazitätssignal ändert sich mit dem Flüssigkeitsniveau in dem Messbehälter 4, so dass das Kapazitätssignal ein Maß der produzierten Menge an Flüssigkeit ausmacht. Bevorzugt beruht die kapazitive Füllstandsmessung auf einer andauernden Kapazitätsmessung zwischen zwei oder mehreren Sensorflächen. Während der Messung besteht zwischen Sensor und Messmedium kein physischer Kontakt, so dass eine kontaktlose kapazitive Füllstandsmessung erzielt wird. Bei Anwesenheit eines Messmediums in ausreichend nahem Abstand zu den Sensorflächen kommt es zu einer Kapazitätsänderung, die bei geeigneter Konzeption des Messgefäßes direkt auf den Füllstand des Messmediums im Messbehälter schließen lässt. Die Kapazität wird hierfür mit einer dafür vorgesehenen Sensorelektronik erfasst, quantisiert und verarbeitet. Über die Dimensionen des Messbehälters 4 kann bei Kenntnis des Füllstandes anschließend auf das Volumen des Messmediums zurückgerechnet werden.

Wie aus Fig. 1 ersichtlich, weist der Grundköper 2 eine Längsführung 19 auf, in welcher der Ableitschlauch 3 für die Körperflüssigkeit aufgenommen ist. Der Messbehälter 4 befindet sich seitlich neben der Längsführung 19 mit dem Ableitschlauch 3. Die Längsführung 19 erstreckt sich über die gesamte Länge des Gehäuses 2 von oben nach unten. In der gezeigten Ausführung weist die Längsführung 19 an einer Längsseite des Gehäuses 2 eine seitliche Einführöffnung 19A auf, durch welche der Ableitschlauch 3 zumindest teilweise im Inneren der Längsführung 19 angeordnet werden kann. Die Einführöffnung 19A erstreckt sich über im Wesentlichen die gesamte Länge der Längsführung 19 von der oberen zur unteren Stirnseite des Gehäuses 2. Neben der Längsführung 19 ist der Messbehälter 4 angeordnet, welcher hier als Messzylinder mit einer im Wesentlichen parallel zur Längsführung 19 verlaufenden Längsachse ausgebildet ist.

Bei der Ausführungsform der Fig. 1 und Fig. 2 sind der Ableitschlauch 3, die Flüssigkeitsverbindung 5 und der Messbehälter 4 als integrale Messeinheit 20 ausgebildet, welche reversibel lösbar mit dem Gehäuse 2 verbunden wird. Zu diesem Zweck weist das Gehäuse 2 die Längsführung 19 für den Ableitschlauch 3, eine Ausnehmung 2A für den Messbehälter 4 und eine (nicht ersichtliche) Aussparung für die Flüssigkeitsverbindung 5 auf. Beispielsweise können bei dieser Ausführung der Ableitschlauch 3, der Messbehälter 4 und die Flüssigkeitsverbindung 5 zwischen dem Messbehälter 4 und dem Ableitschlauch 3 als Wegwerfkomponenten ausgebildet sein. Die Messeinheit 20 mit dem Ableitschlauch 3, der Flüssigkeitsverbindung 5 und dem Messbehälter 4 können in einem Arbeitsschritt am Grundkörper 2 angebracht werden. Benachbart der Längsführung 19, der Ausnehmung 2A und der Aussparung kann das Gehäuse 2 eine elastische Auskleidung 21, beispielsweise aus Silikon oder Polyurethan, aufweisen, welche die Anordnung der Messeinheit 20 erleichtert. Somit kann die Messeinheit 20 bestehend aus Ableitschlauch 3, Flüssigkeitsverbindung 5 und Messbehälter 4 einfach in das Gehäuse 2 eingefügt und daraus wieder entfernt werden. Dies ist dann besonders vorteilhaft, wenn die Anordnung aus Ableitschlauch 3, Flüssigkeitsverbindung 5 und Messbehälter 4 werkseitig als integrale Messeinheit 20 gefertigt ist oder vor dem Einlegen in die Grundkörper 2 aus den Einzelkomponenten zusammengefügt wird.

In der gezeigten Ausführungsform sind zudem weitere Sensoren 27, beispielsweise Lichtschranken oder kapazitive Sensoren, zur Füllstandsüberwachung benachbart der Längsführung 19 für den Ableitschlauch 3 vorgesehen.

Die Messeinrichtung 1 weist zudem eine Steuereinheit auf, mit welcher das erste Ventil 6 und das zweite Ventil 7 abhängig von den Signalen der Sensoreinheit 16 und gegebenenfalls der weiteren Sensoren 27 umgeschaltet werden können. Die Steuereinheit ist zudem mit einer Auswerteinheit verbunden, mit welcher die Gesamtmenge und/oder die Durchflussrate der Körperflüssigkeit bestimmt wird. Darüber hinaus kann eine Anzeigeeinheit zur Anzeige von Messinformationen vorgesehen sein (nicht gezeigt).

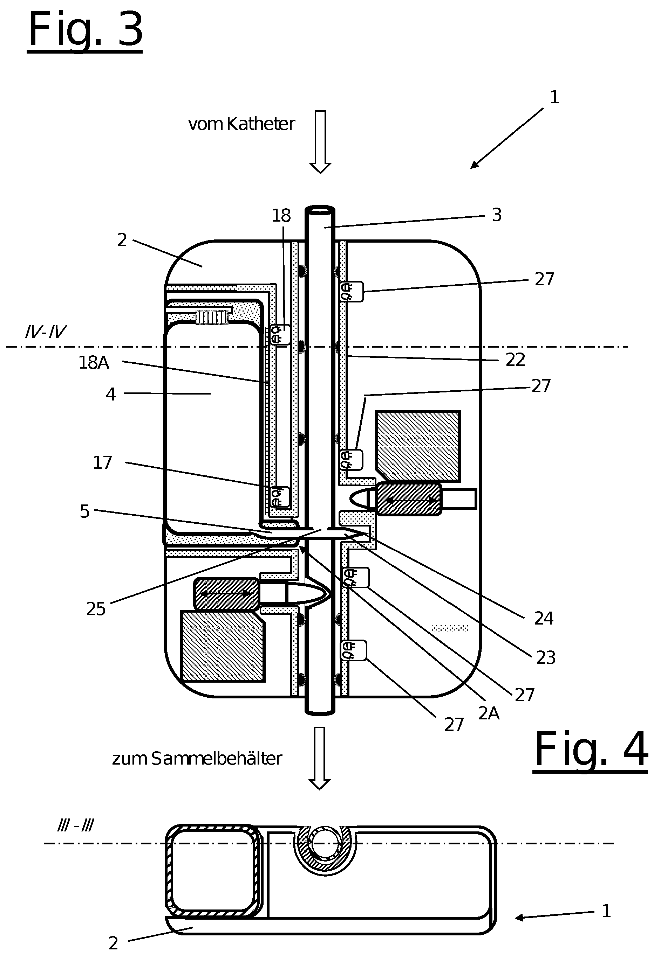

Bei der Ausführungsform der Fig. 3 und Fig. 4 weist die Flüssigkeitsverbindung 5 ein Einstechelement 23 auf, welches dazu eingerichtet ist, den Ableitschlauch 3 zur Herstellung der fluidischen Verbindung zu durchstechen. Zu diesem Zweck ist die Flüssigkeitsverbindung 5 aus einem längssteifen Material gefertigt. Das Einstechelement 23 weist eine Spitze 24 zum Durchdringen des Ableitschlauchs 3 auf. In der gezeigten Variante werden gegenüberliegende Schlauchwände des Ableitschlauchs 3 durchstochen. Das Einstechelement 23 weist (in Einstechrichtung gesehen hinter der Spitze 24) einen Mantel auf, an welchem eine Flüssigkeitseintrittsöffnung 25 vorgesehen ist. Über die Flüssigkeitseintrittsöffnung 25 strömt die Körperflüssigkeit vom Ableitschlauch 3 durch das Innere der Flüssigkeitsverbindung 5 in den Messbehälter 4. Im gezeigten Beispiel wird die fluidische Verbindung vom Ableitschlauch 3 in den Messbehälter 4 beim Einsetzen des Messbehälters 4 in das Gehäuse 2 hergestellt. Zu diesem Zweck kann der Messbehälter 4 mit der Flüssigkeitsverbindung 5 samt dem Einstechelement 23 seitlich in die Ausnehmung 2A und die Aussparung des Gehäuses 2 eingesetzt werden, wodurch das Einstechelement 23 den Ableitschlauch 3 durchdringt und so die Abzweigung der Körperflüssigkeit vom Ableitschlauch 3 in den Messbehälter 4 ermöglicht.

Wie aus Fig. 3 weiters ersichtlich, kann die Sensoreinheit 16 benachbart des Messbehälters 4 einen zusätzlichen Sensor 18A, beispielsweise einen Kapazitätsmessstreifen, aufweisen, welcher in der Lage ist, den Flüssigkeitspegel der Körperflüssigkeit in dem Messbehälter 4 zwischen dem unteren und dem oberen Flüssigkeitspegel zu bestimmen. Besonders bevorzugt sind sowohl der erste 17 und zweite Sensor 18 als auch der weitere Sensor 18A vorgesehen. Dadurch lassen sich Fehler besser erkennen und Fehlfunktionen vermeiden.

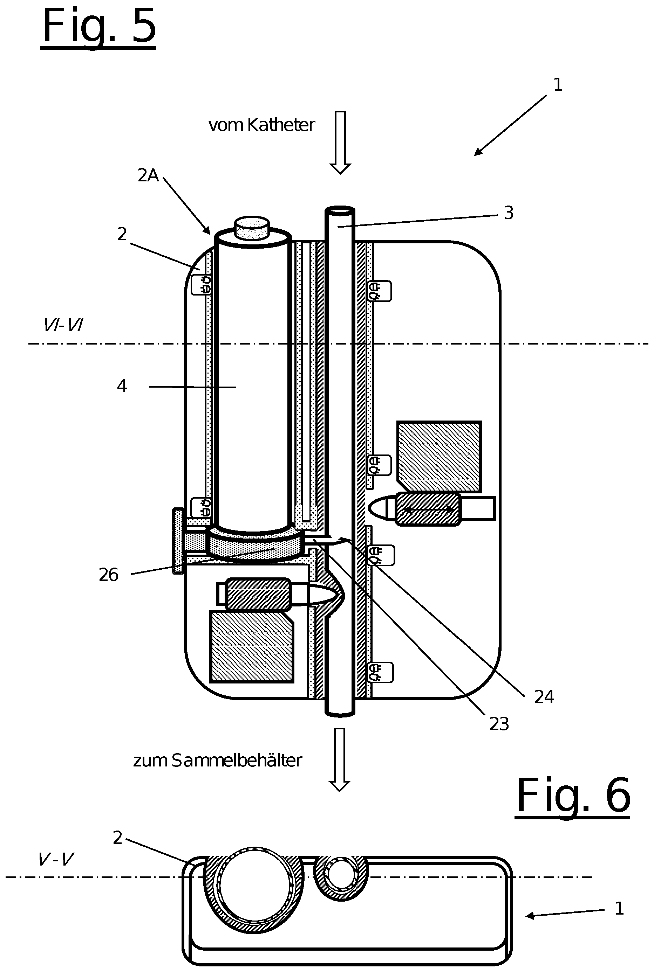

Bei der Ausführungsform der Fig. 5 und Fig. 6 wird die fluidische Verbindung zum Ableitschlauch 3 durch seitliches Einschieben eines mit dem Messbehälter 4 verbundenen Messbehältersockels 26 samt Einstechelement 23 in eine entsprechende Aufnahme des Gehäuses 2 hergestellt. Im gezeigten Beispiel wird der Ableitschlauch 3 einseitig durchstochen, so dass die Spitze 24 im Inneren des Ableitschlauchs 3 angeordnet wird und nicht die gegenüberliegende Schlauchwand durchdringt. Die Flüssigkeitseintrittsöffnung ist an der Spitze 24 des Einstechelements 23 vorgesehen. In diesem Beispiel kann, beispielsweise nach der Einführung des Messbehältersockels 26, der Messbehälter 4 von oben in die entsprechende Ausnehmung 2A des Gehäuses 2 eingeschoben werden. Dadurch wird der Boden 9 des Messbehälters 4 so mit dem Messbehältersockel 26 verbunden, dass die Körperflüssigkeit über die Eintrittsöffnung 8 in den Messbehälter 4 einströmen kann.

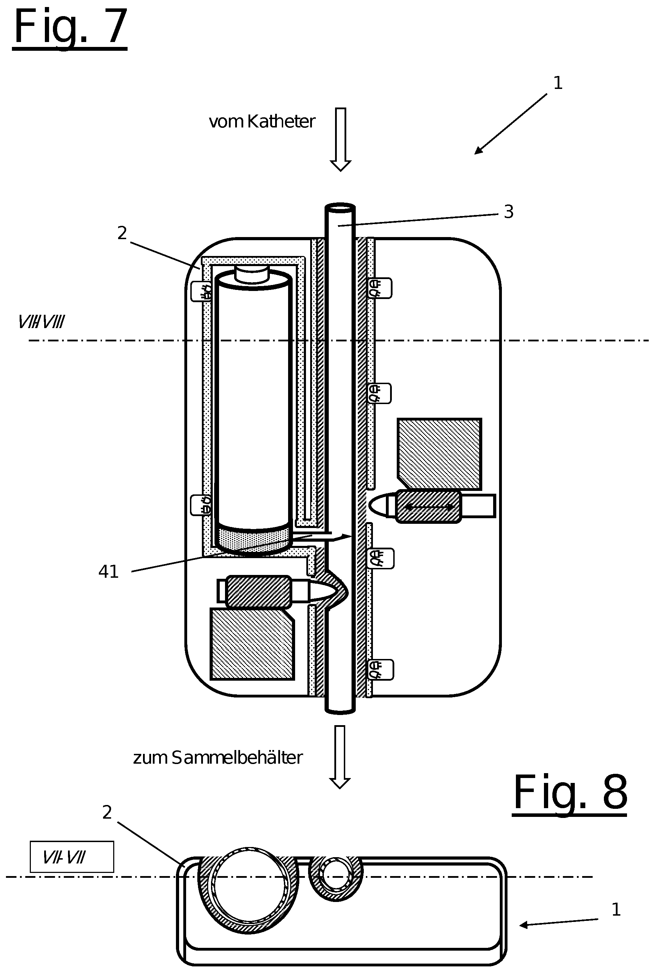

Bei der Ausführungsform der Fig. 7 und Fig. 8 kann die fluidische Verbindung zum Ableitschlauch 3 mithilfe eines Werkzeuges außerhalb des Gehäuses 2 hergestellt werden. Im gezeigten Beispiel wird der Ableitschlauch 3 einseitig durchstochen.

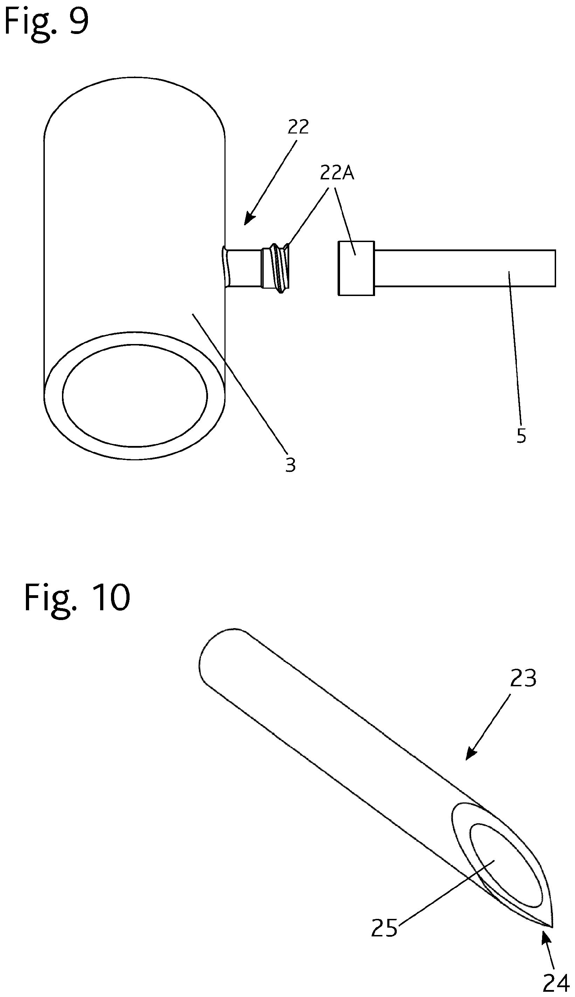

Bei der Ausführungsform der Fig. 9 ist eine (im unverbundenen Zustand dargestellte) Steckverbindung 22, beispielsweise mit Luer-Verbindungsstücken 22A, zur fluidischen Verbindung der Flüssigkeitsverbindung 5 mit dem Ableitschlauch 3 vorgesehen.

Fig. 10 zeigt eine Detailansicht einer Ausführungsform des Einstechelements 23, bei welchem die Flüssigkeitseintrittsöffnung 25 an der Spitze 24 vorgesehen ist.

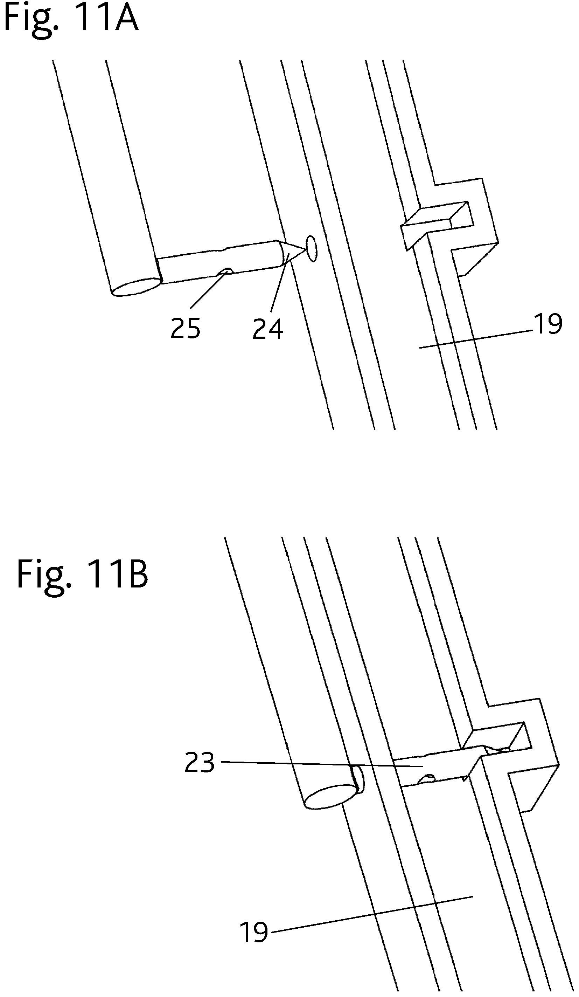

Fig. 11A und Fig. 11B zeigen Detailansichten einer Ausführungsform des Einstechelements 23, bei welchem die Flüssigkeitseintrittsöffnung 25 an einem Mantel hinter der Spitze 24 vorgesehen ist. Durch Einschieben des Einstechelements 23 in die Längsführung 19 kann der (nicht dargestellte) Ableitschlauch 3 zur Herstellung der fluidischen Verbindung mit dem Messbehälter 4 durchstochen werden.

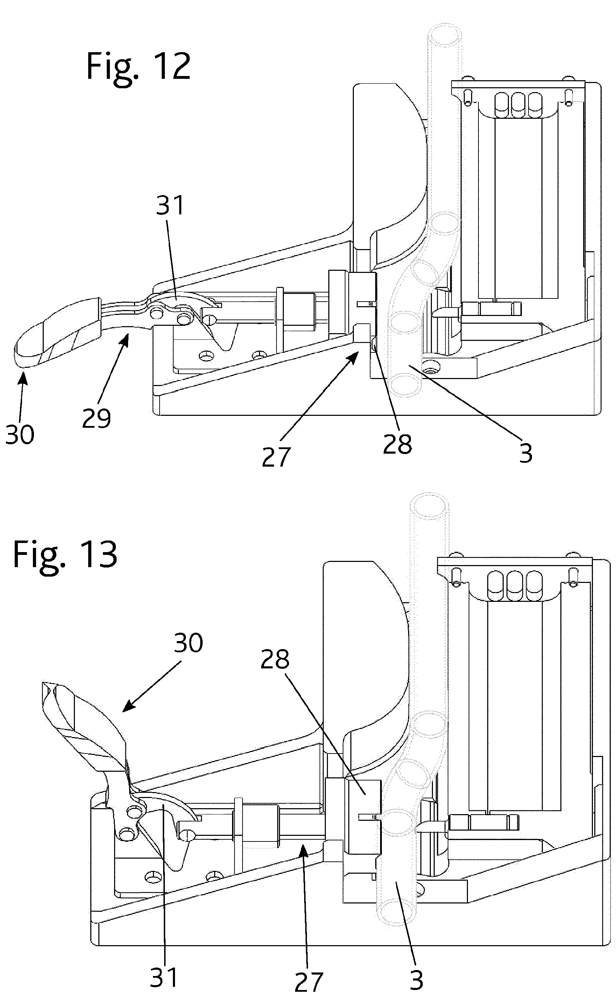

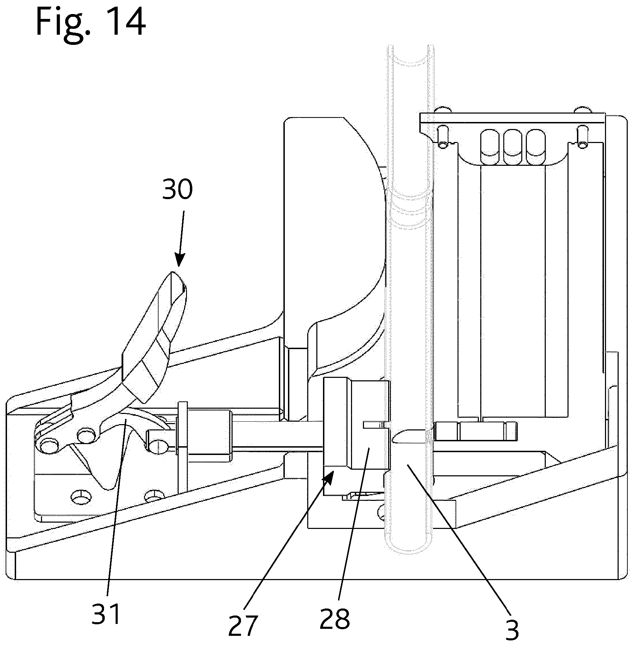

Die Fig. 12 bis Fig. 14 zeigen eine weitere Ausführungsform, bei welcher eine Schubvorrichtung 27 vorgesehen ist, mit welcher der Ableitschlauch 3 derart gegen das Einstechelement 23 gedrückt werden kann, dass der Ableitschlauch 3 eingestochen und über das Einstechelement 23 flüssigkeitsleitend mit dem Messbehälter 4 verbunden wird. Die Schubvorrichtung 27 weist in der gezeigten Ausführung ein Schubelement 28 auf, welches von einer zurückgezogenen Stellung (vgl. Fig. 12) über eine Zwischenstellung (vgl. Fig. 13) in eine vorgeschobene Stellung (vgl. Fig. 14) bewegt werden kann. Dafür weist die Schubvorrichtung 27 einen Hebelmechanismus 29 auf, welcher zwischen einer ersten Position entsprechend der zurückgezogenen Stellung des Schubelements 28 (vgl. Fig. 12) und einer zweiten Position entsprechend der vorgeschobenen Stellung des Schubelements 28 (vgl. Fig. 14) beweglich ist. Der Hebelmechanismus 29 weist einen Schwenkhebel 30 auf, dessen freies Ende als Handgriff für den Benutzer ausgebildet ist. Der Schwenkhebel 30 ist über ein Zwischenelement 31 mit dem Schubelement 28 verbunden, um die Schwenkbewegung des Schwenkhebels 30 über das Zwischenelement 31 in die Verschiebung des Schubelements 28 umzuwandeln. Durch Betätigen des Hebelmechanismus 29 über den Schwenkhebel 30 wird der Ableitschlauch 3 vom Schubelement 28 auf das Einstechelement 23 aufgeschoben, so dass der Ableitschlauch 3 vom Einstechelement 23 aufgestochen wird.

### Bezugsziffernliste:

1 Messeinrichtung
2 Grundkörper
2A Ausnehmung des Gehäuses
3 Ableitschlauch
3A erster Längsabschnitt des Ableitschlauchs
3B zweiter Längsabschnitt des Ableitschlauchs
4 Messbehälter
4A Belüftungsventil
5 Flüssigkeitsverbindung
6 erstes Ventil
7 zweites Ventil
8 Eintrittsöffnung
9 Boden
10 erster Antrieb
11 erstes Getriebe
12 erster Ventilkörper
13 zweiter Antrieb
14 zweites Getriebe
15 zweiter Ventilkörper
16 Sensoreinheit
17 erster Sensor
18 zweiter Sensor
19 Längsführung
19A Einführöffnung der Längsführung
20 Messeinheit
21 Auskleidung
22 Verbindungsstück
22A T-Stück
23 Einstechelement
24 Spitze
25 Flüssigkeitseintrittsöffnung
26 Messbehältersockel
27 Schubvorrichtung
28 Schubelement
29 Hebelmechanismus
30 Schwenkhebel
31 Zwischenelement

## Patentansprüche

1. Messeinrichtung (1) zur Messung der Ausscheidung einer Körperflüssigkeit, insbesondere von Urin, eines Patienten, insbesondere mit einem Katheter, aufweisend:
ein Gehäuse (2),
einen Messbehälter (4) zur Aufnahme eines Messvolumens der Körperflüssigkeit,
eine Flüssigkeitsverbindung (5) zum Zuführen des Messvolumens der Körperflüssigkeit von einem Ableitschlauch (3) in den Messbehälter (4),
ein erstes Ventil (6) zum Freigeben und Blockieren des Durchtritts der Körperflüssigkeit durch einen ersten Längsabschnitt (3A) des Ableitschlauchs (3),
eine Sensoreinheit (16) zur Erfassung des Messvolumens der Körperflüssigkeit in dem Messbehälter (4),
**dadurch gekennzeichnet, dass** die Messeinrichtung (1) ein zweites Ventil (7) zum Freigeben und Blockieren des Durchtritts der Körperflüssigkeit durch einen zweiten Längsabschnitt (3B) des Ableitschlauchs (3) aufweist, und dass
der Messbehälter (4) seitlich zu einer Längsführung (19) für den Ableitschlauch (3) versetzt angeordnet ist,
wobei die Flüssigkeitsverbindung (5) zur Abzweigung des Messvolumens der Körperflüssigkeit zwischen dem ersten (6) und dem zweiten Ventil (6) vom Ableitschlauch (3) in den Messbehälter (4) eingerichtet ist.

2. Messeinrichtung (1) nach Anspruch 1, **dadurch gekennzeichnet, dass** die Flüssigkeitsverbindung (5) zudem zum Ableiten des Messvolumens der Körperflüssigkeit vom Messbehälter (4) in den Ableitschlauch (3) eingerichtet ist.

3. Messeinrichtung (1) nach Anspruch 1 oder 2, **dadurch gekennzeichnet, dass** die Flüssigkeitsverbindung (5) mit einer Eintrittsöffnung (8) am unteren Bereich des Messbehälters (4), insbesondere am Boden (9), des Messbehälters (4) verbunden ist.

4. Messeinrichtung (1) nach einem der Ansprüche 1 bis 3, **dadurch gekennzeichnet, dass** die Längsführung (19) eine Einführöffnung (19A) zum Einführen des Ableitschlauchs (3) aufweist, wobei sich die Einführöffnung (19A) bevorzugt über im Wesentlichen die gesamte Länge der Längsführung (19) erstreckt.

5. Messeinrichtung (1) nach einem der Ansprüche 1 bis 4, **dadurch gekennzeichnet, dass** sich der Messbehälter (4) im Wesentlichen parallel zur Längsführung (19) erstreckt.

6. Messeinrichtung (1) nach einem der Ansprüche 1 bis 5, **dadurch gekennzeichnet, dass** der Ableitschlauch (3) vorgesehen ist, wobei der Ableitschlauch (3), die Flüssigkeitsverbindung (5) und der Messbehälter (4) als Messeinheit (20) ausgebildet sind, welche über eine lösbare Verbindung, insbesondere eine Steckverbindung, mit dem Gehäuse (2) verbunden ist.

7. Messeinrichtung (1) nach einem der Ansprüche 1 bis 5, **dadurch gekennzeichnet, dass** die Flüssigkeitsverbindung (5) ein Verbindungsstück (22) zur reversibel lösbaren Verbindung mit dem Ableitschlauch (3), insbesondere mit einem T-Stück (22A) zwischen dem ersten (3A) und zweiten Längsabschnitt (3B) des Ableitschlauchs (3), aufweist.

8. Messeinrichtung (1) nach einem der Ansprüche 1 bis 5, **dadurch gekennzeichnet, dass** die Flüssigkeitsverbindung (5) ein Einstechelement (23) aufweist, welches zum Einstechen des Ableitschlauchs (3), insbesondere im Wesentlichen senkrecht zur Längsführung (19), zwischen dem ersten (6) und dem zweiten Ventil (7) eingerichtet ist.

9. Messeinrichtung (1) nach einem der Ansprüche 1 bis 8, **dadurch gekennzeichnet, dass** der Messbehälter (4), insbesondere an der Oberseite, ein Belüftungsventil (4A) aufweist.

10. Verfahren zur Messung der Ausscheidung einer Körperflüssigkeit, insbesondere von Urin, eines Patienten, insbesondere mit einem Katheter, mit den Schritten:
Verbinden einer Messeinrichtung (1) nach einem der Ansprüche 1 bis 9 mit einem Ableitschlauch (3), wobei der Ableitschlauch (3) in der Längsführung (19) des Gehäuses (2) angeordnet wird,
Schalten des ersten Ventils (6) in eine Offenstellung und des zweiten Ventils (7) in eine Schließstellung,
Abzweigen eines Messvolumens der Körperflüssigkeit vom Ableitschlauch (3) über die Flüssigkeitsverbindung (5) in den Messbehälter (4),
Erfassung des Messvolumens der Körperflüssigkeit in dem Messbehälter (4).

11. Verfahren nach Anspruch 10, **gekennzeichnet durch**:
Schalten des ersten Ventils (6) in die Schließstellung und des zweiten Ventils (7) in die Offenstellung,
Entleeren des Messvolumens der Körperflüssigkeit über die Flüssigkeitsverbindung (5).

12. Verfahren nach Anspruch 10, **gekennzeichnet durch**:
Schalten des ersten Ventils (6) in eine Offenstellung und Schalten des zweiten Ventils (7) in eine Offenstellung,
Ableiten der Körperflüssigkeit durch den Ableitschlauch (3) vorbei an dem Messbehälter (4).

## Claims

1. Measuring device (1) for measuring the excretion of a body fluid, in particular of urine, of a patient, in particular with
a catheter, comprising:
a housing (2),
a measuring container (4) for receiving a measuring volume of the body fluid,
a fluid connection (5) for supplying the measuring volume of the body fluid from a drainage tube (3) into the measuring container (4),
a first valve (6) for releasing and blocking the passage of the body fluid through a first longitudinal section (3A) of the drainage tube (3),
a sensor unit (16) for detecting the measuring volume of the body fluid in the measuring container (4),
**characterized in that** the measuring device (1) comprises a second valve (7) for releasing and blocking the passage of the body fluid through a second longitudinal section (3B) of the drainage tube (3), and that
the measuring container (4) is arranged laterally offset to a longitudinal guide (19) for the drainage tube (3),
wherein the fluid connection (5) is configured for branching off the measuring volume of the body fluid between the first (6) and the second valve (7) from the drainage tube (3) into the measuring container (4).

2. Measuring device (1) according to claim 1, **characterized in that** the fluid connection (5) is additionally configured for discharging the measuring volume of the body fluid from the measuring container (4) into the drainage tube (3).

3. Measuring device (1) according to claim 1 or 2, **characterized in that** the fluid connection (5) is connected to an inlet opening (8) at the lower region of the measuring container (4), in particular at the bottom (9) of the measuring container (4).

4. Measuring device (1) according to any one of claims 1 to 3, **characterized in that** the longitudinal guide (19) comprises an insertion opening (19A) for inserting the drainage tube (3), wherein
the insertion opening (19A) preferably extends over substantially the entire length of the longitudinal guide (19).

5. Measuring device (1) according to any one of claims 1 to 4, **characterized in that** the measuring container (4) extends substantially parallel to the longitudinal guide (19).

6. Measuring device (1) according to any one of claims 1 to 5, **characterized in that** the drainage tube (3) is provided, wherein the drainage tube (3), the fluid connection (5) and the measuring container (4) are formed as a measuring unit (20) which is connected to the housing (2) via a detachable connection, in particular a plug-in connection.

7. Measuring device (1) according to any one of claims 1 to 5, **characterized in that** the fluid connection (5) comprises a connecting piece (22) for the reversibly detachable connection with the drainage tube (3), in particular with a T-piece (22A) between the first (3A) and second longitudinal section (3B) of the drainage tube (3).

8. Measuring device (1) according to any one of claims 1 to 5, **characterized in that** the fluid connection (5) comprises a piercing element (23) which is configured for piercing the drainage tube (3), in particular substantially perpendicular to the longitudinal guide (19), between the first (6) and the second valve (7).

9. Measuring device (1) according to any one of claims 1 to 8, **characterized in that** the measuring container (4) comprises a ventilation valve (4A), in particular on the upper side.

10. Method for measuring the excretion of a body fluid, in particular of urine, of a patient, in particular with a catheter, comprising the steps:
Connecting a measuring device (1) according to any one of claims 1 to 9 to a drainage tube (3), wherein the drainage tube (3) is arranged in the longitudinal guide (19) of the housing (2),
Switching the first valve (6) into an open position and the second valve (7) into a closed position,
Branching off a measuring volume of the body fluid from the drainage tube (3) via the fluid connection (5) into the measuring container (4),
Detecting the measuring volume of the body fluid in the measuring container (4).

11. Method according to claim 10, **characterized by**:
Switching the first valve (6) into the closed position and the second valve (7) into the open position,
Emptying the measuring volume of the body fluid via the fluid connection (5).

12. Method according to claim 10, **characterized by**:
Switching the first valve (6) into an open position and switching the second valve (7) into an open position,
Discharging the body fluid through the drainage tube (3) past the measuring container (4).

## Revendications

1. Dispositif de mesure (1) pour la mesure de l'excrétion d'un liquide corporel, en particulier d'urine, d'un patient, en particulier avec un cathéter, comprenant :
un boîtier (2),
un récipient de mesure (4) pour la réception d'un volume de mesure du liquide corporel,
une liaison fluidique (5) pour amener le volume de mesure du liquide corporel depuis un tuyau d'évacuation (3) dans le récipient de mesure (4),
une première vanne (6) pour libérer et bloquer le passage du liquide corporel à travers un premier tronçon longitudinal (3A) du tuyau d'évacuation (3),
une unité de capteur (16) pour la détection du volume de mesure du liquide corporel dans le récipient de mesure (4),
**caractérisé en ce que** le dispositif de mesure (1) comprend une deuxième vanne (7) pour libérer et bloquer le passage du liquide corporel à travers un deuxième tronçon longitudinal (3B) du tuyau d'évacuation (3), et **en ce que**
le récipient de mesure (4) est disposé décalé latéralement par rapport à un guide longitudinal (19) pour le tuyau d'évacuation (3),
la liaison fluidique (5) étant configurée pour la dérivation du volume de mesure du liquide corporel entre la première (6) et la deuxième vanne (7) depuis le tuyau d'évacuation (3) dans le récipient de mesure (4).

2. Dispositif de mesure (1) selon la revendication 1, **caractérisé en ce que** la liaison fluidique (5) est en outre configurée pour évacuer le volume de mesure du liquide corporel depuis le récipient de mesure (4) dans le tuyau d'évacuation (3).

3. Dispositif de mesure (1) selon la revendication 1 ou 2, **caractérisé en ce que** la liaison fluidique (5) est reliée à une ouverture d'entrée (8) au niveau de la zone inférieure du récipient de mesure (4), en particulier au niveau du fond (9), du récipient de mesure (4).

4. Dispositif de mesure (1) selon l'une des revendications 1 à 3, **caractérisé en ce que** le guide longitudinal (19) comprend une ouverture d'introduction (19A) pour l'introduction du tuyau d'évacuation (3), l'ouverture d'introduction (19A) s'étendant de préférence sur sensiblement toute la longueur du guide longitudinal (19).

5. Dispositif de mesure (1) selon l'une des revendications 1 à 4, **caractérisé en ce que** le récipient de mesure (4) s'étend sensiblement parallèlement au guide longitudinal (19).

6. Dispositif de mesure (1) selon l'une des revendications 1 à 5, **caractérisé en ce que** le tuyau d'évacuation (3) est prévu, le tuyau d'évacuation (3), la liaison fluidique (5) et le récipient de mesure (4) étant réalisés sous la forme d'une unité de mesure (20), laquelle est reliée au boîtier (2) par l'intermédiaire d'une liaison amovible, en particulier une liaison enfichable.

7. Dispositif de mesure (1) selon l'une des revendications 1 à 5, **caractérisé en ce que** la liaison fluidique (5) comprend une pièce de raccordement (22) pour la liaison amovible de manière réversible avec le tuyau d'évacuation (3), en particulier avec une pièce en T (22A) entre le premier (3A) et le deuxième tronçon longitudinal (3B) du tuyau d'évacuation (3).

8. Dispositif de mesure (1) selon l'une des revendications 1 à 5, **caractérisé en ce que** la liaison fluidique (5) comprend un élément de perçage (23), lequel est configuré pour percer le tuyau d'évacuation (3), en particulier sensiblement perpendiculairement au guide longitudinal (19), entre la première (6) et la deuxième vanne (7).

9. Dispositif de mesure (1) selon l'une des revendications 1 à 8, **caractérisé en ce que** le récipient de mesure (4) comprend, en particulier sur le côté supérieur, une vanne de ventilation (4A).

10. Procédé pour la mesure de l'excrétion d'un liquide corporel, en particulier d'urine, d'un patient, en particulier avec un cathéter, comprenant les étapes suivantes :
Relier un dispositif de mesure (1) selon l'une des revendications 1 à 9 à un tuyau d'évacuation (3), le tuyau d'évacuation (3) étant disposé dans le guide longitudinal (19) du boîtier (2),
Commuter la première vanne (6) dans une position d'ouverture et la deuxième vanne (7) dans une position de fermeture,
Dériver un volume de mesure du liquide corporel depuis le tuyau d'évacuation (3) via la liaison fluidique (5) dans le récipient de mesure (4),
Détecter le volume de mesure du liquide corporel dans le récipient de mesure (4).

11. Procédé selon la revendication 10, **caractérisé par** :
Commuter la première vanne (6) dans la position de fermeture et la deuxième vanne (7) dans la position d'ouverture,
Vider le volume de mesure du liquide corporel via la liaison fluidique (5).

12. Procédé selon la revendication 10, **caractérisé par** :
Commuter la première vanne (6) dans une position d'ouverture et commuter la deuxième vanne (7) dans une position d'ouverture,
Évacuer le liquide corporel à travers le tuyau d'évacuation (3) en contournant le récipient de mesure (4).
